# FASCICULE DE BREVET EUROPEEN

(11) **EP 4 093 266 B1**
(45) Date de publication et mention de la délivrance du brevet: **23.04.2025**
(21) Numéro de dépôt: 21705593.8
(22) Date de dépôt: 21.01.2021
(51) Int. Cl.: A61B 5/00, G01N 3/14, G06T 7/50, G01N 3/06

(54) **SYSTEME ET PROCEDE DE MESURE D'UNE FORCE EXERCEE SUR UN CORPS, ET CARACTERISATION DE LA RIGIDITE D'UN CORPS EMPLOYANT UN TEL DISPOSITIF**
SYSTEM UND VERFAHREN ZUR MESSUNG EINER AUF EINEN KÖRPER AUSGEÜBTEN KRAFT UND ZUR CHARAKTERISIERUNG DER STEIFIGKEIT EINES KÖRPERS MIT EINER SOLCHEN VORRICHTUNG
SYSTEM AND METHOD FOR MEASURING A FORCE EXERTED ON A BODY, AND FOR CHARACTERISING THE RIGIDITY OF A BODY USING SUCH A DEVICE

(30) Priorité: 23.01.2020 FR 2000652
(43) Date de publication de la demande: 30.11.2022
(73) Titulaire: Anatoscope, 34960 Montpellier (FR)
(72) Inventeur: FAURE, François, 38920 CROLLES (FR)
(74) Mandataire: IP Trust
(86) Numéro de dépôt international: PCT/FR2021/050108
(87) Numéro de publication internationale: WO 2021/148755

(56) Documents cités:
- WO-A1-2018/029286
- US-A1- 2011 054 354
- US-A1- 2011 319 791
- BERND BICKEL ET AL: "Capture and modeling of non-linear heterogeneous soft tissue", ACM TRANSACTIONS ON GRAPHICS, ACM, NY, US, vol. 28, no. 3, 27 July 2009 (2009-07-27), pages 1 - 9, XP058145407, ISSN: 0730-0301, DOI: 10.1145/1531326.1531395

## Description

### DOMAINE DE L'INVENTION

La présente invention concerne un système et un procédé de mesure d'une force exercée sur un corps. Ce système et ce procédé trouvent leurs applications dans le domaine de la caractérisation et de la modélisation de la rigidité d'un corps.

### ARRIERE PLAN TECHNOLOGIQUE DE L'INVENTION

Il existe une multitude de situations dans lesquelles il est utile de disposer d'une caractérisation de la rigidité d'un corps.

Dans le domaine médical, il est courant d'examiner un patient par palpation, c'est-à-dire par contact physique direct. La distribution de la rigidité dans la partie du corps ausculté permet au praticien d'identifier des régions du corps du patient susceptibles d'être anormales, comportant par exemple des tumeurs.

La mesure de la rigidité d'une partie d'un corps humain peut trouver d'autres applications, par exemple pour concevoir un objet destiné à être porté par un utilisateur (par exemple une prothèse, une orthèse, voire même un vêtement) qui est non seulement ajusté à la morphologie de l'utilisateur, mais qui prend également en compte la déformation du corps de l'utilisateur lorsque la prothèse, l'orthèse ou le vêtement est disposé sur le corps de l'utilisateur et exerce une pression sur ce corps.

La mesure de la rigidité est également utile en vue de la modélisation complète d'un corps déformable (un corps humain ou un corps de toute autre nature), le modèle représentant alors le comportement de déformation de ce corps lorsque celui-ci est soumis à des efforts extérieurs. Le modèle peut être utile pour permettre la réplication fidèle de cet objet.

Comme le rappelle le document « Capture and modeling of non linear heterogeneous soft tissue », de B. Bickel et al. ACM Transactions on Graphics 28(3):89, July 2009, le travail de caractérisation et/ou de modélisation d'un corps déformable peut mettre en œuvre un système de mesure de la déformation du corps lorsque celui-ci est soumis à un effort. Plus spécifiquement, le système de mesure proposé par ce document comprend une sonde de contact pour exercer ponctuellement un effort sur le corps. La sonde comporte un capteur d'effort pour mesurer l'effort appliqué par la partie distale d'une tige rigide munie d'un embout d'application. Le système comprend également 3 dispositifs de prises de vue permettant d'enregistrer la déformation de la surface du corps sous trois angles différents, lorsque la sonde exerce un effort sur celui-ci. L'activation d'un déclencheur par l'opérateur de la sonde de contact permet de procéder simultanément à la mesure de la force appliquée et aux 3 prises de vues. Des marqueurs sont peints sur le corps et sur la tige de la sonde afin de permettre de repérer, par traitement d'images, la déformation de la surface et la position/orientation de la sonde.

Un système de mesure similaire est décrit dans le document US9623608. Ce système comprend un bras de robot muni d'un capteur de force, le bras étant configuré pour appliquer un effort sur le corps ou l'objet sous test. Le système comprend également une pluralité de dispositifs de prise de vues, disposés en demi-cercle au-dessus du bras du robot, pour suivre la déformation de l'objet au cours de la séquence de mesure. L'objet peut porter des moyens pour assister le traitement d'images réalisées par le système de mesure.

Le document US 2011/0319791 divulgue un système adapté pour la mesure des propriétés mécaniques d'un matériau déformable. Le système déforme la surface du matériau à l'aide d'une sonde, telle qu'une sonde flexible, tout en enregistrant optiquement en détail la topographie tridimensionnelle de la déformation de la surface résultante.

Le document "The human touch: Measuring contact with real human soft tissues" de Pai et al, ACM Transactions on Graphics. 37. 1-12. (2018) propose une sonde de contact portable comprenant une tête portant trois caméras RGB et une tige rigide, reliée à la tête, associée à un capteur de force et de couple. L'extrémité distale de la tige est destinée à entrer en contact avec le corps d'un patient pour appliquer une force et la déformer. Un système de suivi capte des images par l'intermédiaire de trois caméras disposées dans la pièce, pour permettre de repérer et suivre la sonde dans un repère lié au monde. Ces images sont traitées numériquement pour établir un flux optique (i.e. le mouvement apparent du corps sous l'effet de l'application de la force) ce qui permet d'analyser le déplacement de la surface de la peau du patient.

Le document de Faragasso et al "Real-Time Vision-Based Stiffness Mapping" (2018), Sensors, 18, 1347 décrit un dispositif de mesure de la déformation d'un corps ou d'un objet. Le dispositif comprend une pluralité de poinçons, respectivement montés sur des ressorts et reliés à une base. Lorsqu'on applique l'extrémité des poinçons contre l'objet ou le corps, les ressorts se contractent selon la force appliquée, leurs raideurs et la rigidité du corps de l'objet au niveau des points respectifs de contact. Le dispositif de mesure est muni d'un dispositif de prise de vue permettant de capter une image des ressorts pour repérer leurs contractions et ainsi déterminer la déformation du corps au niveau de chaque point de contact avec les poinçons. La résolution d'un système d'équations non linéaires permet de déterminer la raideur de l'objet, en faisant l'hypothèse que cette raideur est la même sous chaque poinçon.

Ces dispositifs de l'état de la technique sont particulièrement complexes à mettre en œuvre. Certains d'entre eux nécessitent d'identifier la position et l'orientation de la sonde de contact sur les images fournies par les caméras pour reconstituer toutes les composantes de la force exercée sur le corps. Le dispositif décrit par le document de Faragasso impose d'appliquer l'effort dans la direction de contraction des ressorts, cette solution ne prend pas en compte l'existence de composantes tangentielles de l'effort appliqué. Par ailleurs, l'application de l'effort est réalisée sur une zone du corps relativement étendue, ce qui ne permet pas de bien localiser le point d'application de cet effort et limite la résolution de l'analyse de rigidité.

Un but de la présente invention est de remédier, au moins en partie, aux limitations de cet état de la technique. Elle vise en particulier à fournir un système de mesure d'une force exercée sur un corps qui soit simple à mettre en œuvre. Ce système peut être utilisé pour caractériser la rigidité d'un corps avec une résolution satisfaisante.

### BREVE DESCRIPTION DE L'INVENTION

En vue de la réalisation de l'un de ces buts, l'objet de l'invention propose un système de mesure destiné à mesurer une force exercée sur un corps, le système de mesure comprend un dispositif de mesure et des moyens de calcul,
le dispositif de mesure comprend :
   - une tige flexible présentant une extrémité proximale et une extrémité distale destinée à entrer en contact avec le corps et à appliquer une force tendant à le déformer au voisinage d'un point de contact, la tige flexible étant configurée pour fléchir dès lors qu'elle applique une force par son extrémité distale sur le corps, la tige flexible est courbée ou présente un coude;
   - un dispositif de prise de vue solidaire de l'extrémité proximale et orientée vers l'extrémité distale de la tige flexible pour fournir au moins une carte de profondeur du corps au voisinage de l'extrémité distale de la tige flexible, et de l'extrémité distal ;
les moyens de calcul sont configurés pour, à partir de la carte de profondeur de l'extrémité distale de la tige, déterminer la force exercée par la tige flexible par son extrémité distale sur le corps.

Selon un mode de réalisation, la tige flexible est configurée pour appliquer une force comprise entre 0,1 N et 10 N.

Selon un mode de réalisation, le dispositif de mesure comprend un interrupteur pour actionner le dispositif de prise de vue.

Selon un mode de réalisation, le système comprend en outre une poignée de préhension solidaire du dispositif de prise de vue et de la tige flexible.

Selon un mode de réalisation, l'interrupteur se présente sous la forme d'une gâchette.

Selon un mode de réalisation, les moyens de calcul comprennent une unité de transmission et un dispositif de calcul distant, l'unité de transmission étant configurée pour échanger des données avec un dispositif de calcul distant.

Selon un mode de réalisation, les moyens de calcul comprennent un unité de calcul configurée pour traiter la carte de profondeur.

Selon un mode de réalisation, le dispositif de prise de vue fournit également une image numérique du corps au voisinage de l'extrémité distale de la tige flexible.

Selon un mode de réalisation, l'extrémité distale comporte un embout présentant une couleur pour être facilement repérée sur l'image fournie par le dispositif de prise de vue.

Selon un mode de réalisation, l'extrémité distale comporte un embout présentant une forme choisie pour être facilement repérée sur la carte fournie par le dispositif de prise de vue.

L'invention concerne également un procédé de mesure d'une force exercée sur un corps, le procédé comprenant :
- une étape d'application du système de mesure selon la présente invention pour que l'extrémité distale de la tige contacte le corps en un point de contact et applique une force tendant à déformer le corps au voisinage du point de contact ;
- une étape d'acquisition, à un instant de mesure, d'au moins une carte de profondeur au cours de l'étape d'application ;
- une étape de traitement de la carte de profondeur pour établir la force exercée sur le corps.

Selon un mode de réalisation, l'étape de traitement comprend une sous-étape de repérage de l'extrémité distale de la tige dans la carte de profondeur pour fournir des coordonnées de cette extrémité dans un repère associé au dispositif de mesure.

Selon un mode de réalisation, dans lequel le procédé comprend une étape préalable de calibration du dispositif de mesure.

L'invention concerne également un procédé de caractérisation de la rigidité d'un corps comprenant l'acquisition, au cours d'un procédé de mesure conforme à la présente invention, d'une pluralité de cartes de profondeur en des instants de mesures successifs avec un système selon l'invention.

Selon un mode de réalisation, le procédé de caractérisation comprend une étape de recalage de la pluralité de cartes de profondeur.

Selon un mode de réalisation, le corps comprend des repères de recalage permettant de recaler la pluralité de cartes de déformation.

### BREVE DESCRIPTION DES DESSINS

D'autres caractéristiques et avantages de l'invention ressortiront de la description détaillée de l'invention qui va suivre en référence aux figures annexées sur lesquels :
La figure 1 représente un système de mesure de la force exercée sur un corps conforme à l'invention.
Les figures 2a, 2b et 2c représentent schématiquement et respectivement le comportement du dispositif de mesure avant le contact, en simple contact et en contact forcé avec un corps sous test ;
Les figures 3a à 3c représentent un exemple des traitements mis en œuvre au cours d'un procédé conforme à l'invention ;
Les figures 4a à 4c représentent un exemple des traitements mis en œuvre au cours d'un procédé de caractérisation de la rigidité d'un corps conforme à un aspect de l'invention.

### DESCRIPTION DETAILLEE DE L'INVENTION

### Système de mesure de la force exercée sur un corps

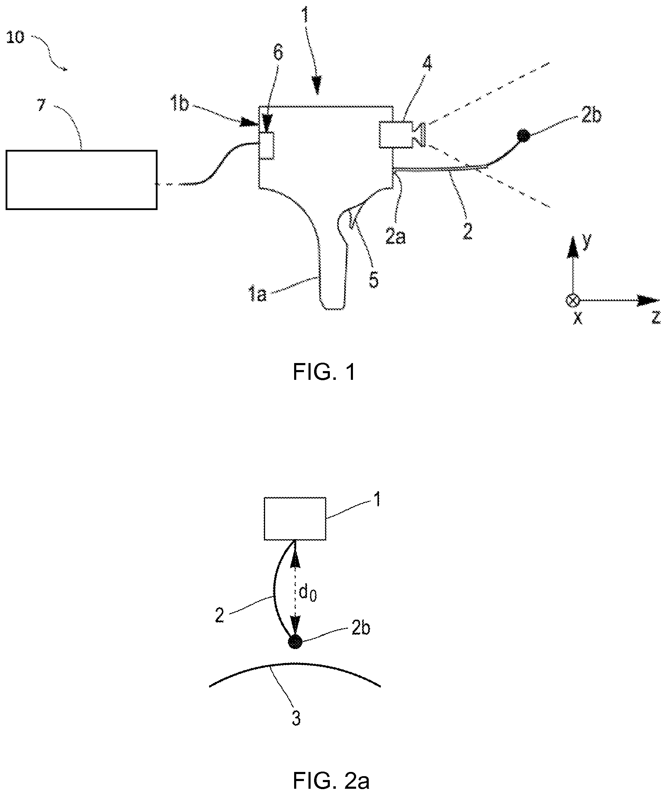

Dans l'exemple représenté sur la figure 1, un système de mesure 10 qui comprend un dispositif de mesure 1 et des moyens de calcul 7. Le dispositif de mesure 1 conforme à l'invention est formé d'une tête 1b et d'une poignée de préhension 1a. La tête 1b porte une tige flexible 2 présentant une extrémité proximale à laquelle la tête 1b est reliée. Une extrémité distale 2b de la tige flexible 2 est quant à elle destinée à entrer en contact avec un corps sous test. Plus précisément, le dispositif de mesure 1 est manipulé par un opérateur pour approcher l'extrémité distale 2b du corps, de manière à entrer en contact avec lui. On transmet et on applique de la sorte une force au corps tendant à le déformer au voisinage du point de contact.

Pour des raisons de sécurité, ainsi que pour éviter d'endommager le corps au niveau du point de contact, on peut prévoir de munir l'extrémité distale 2b d'un embout de protection. Celui-ci peut présenter une surface de contact, c'est-à-dire une surface destinée à entrer en contact avec le corps, plane ou arrondie de manière à assurer cette fonction de protection, sans toutefois être de trop grande dimension pour préserver la nature ponctuelle du contact qui se forme entre l'extrémité distale 2b de la tige flexible 2 et le corps sous test.

À titre d'exemple, l'embout de protection peut correspondre à une sphère en plastique enchâssée sur l'extrémité distale 2b de la tige flexible, et présentant un diamètre de l'ordre de quelques millimètres, par exemple compris entre 2mm et 5mm. Comme cela sera détaillé dans la suite de cet exposé, la forme de l'embout et/ou sa couleur peut être également choisie pour être facilement repérée sur une carte de profondeur ou une image dont on fait l'acquisition au cours de l'utilisation du dispositif de mesure 1.

Ainsi que cela a déjà été mentionné, la tige 2 permet de transmettre une force appliquée au dispositif de mesure 1 et d'appliquer cette force sur le corps. Cette tige 2 est flexible, c'est-à-dire que la force de réaction qui s'applique sur l'extrémité distale 2b de la tige 2 lorsque celle-ci est en contact forcé sur le corps tend à la déformer, par exemple par flexion. Dans une certaine mesure, la flexibilité de la tige 2 permet de rendre indépendant le déplacement de l'extrémité distale 2b de la tige du déplacement de la tête 1b du dispositif de mesure 1 sur laquelle elle est fixée. Le matériau composant la tige flexible 2 et sa forme sont choisis pour que, dans la gamme des efforts que l'on cherche à appliquer sur le corps, la tige 2 puisse se déformer élastiquement. Lorsque le corps est un corps humain, cet effort peut être compris entre 0,1 N et 10 N.

On peut ainsi prévoir que la tige 2 soit formée d'un métal ou d'un plastique. Selon l'invention, elle présente un coude ou est incurvée (comme cela est représenté sur la figure 1) pour favoriser sa flexion élastique lors de l'application du dispositif de mesure 1 sur le corps sous test. La forme courbée de la tige 2 peut également présenter l'avantage de placer l'extrémité distale 2b, lorsque la tige 2 n'est pas déformée, au centre (ou à proximité du centre) du champ de vision d'un dispositif de prise de vue 4 du dispositif de mesure 1, comme cela sera détaillé dans la suite de cette description.

Poursuivant la description de la figure 1, le dispositif de mesure 1 comprend également un dispositif de prise de vue 4, ici une caméra intégrée à la tête 1b du dispositif 1. La tête 1b permet de figer les positions relatives du dispositif de prise de vue 4 et de l'extrémité proximale 2a de la tige 2 de manière à les rendre solidaires l'un de l'autre. La tête 1b ne forme toutefois pas un élément essentiel du dispositif de mesure 1 et l'on pourrait envisager d'autres configurations de ce dispositif 1 dans lequel le dispositif de prise de vue 4 est solidaire de l'extrémité proximale 2a de la tige 2 sans nécessiter la présence d'une tête. On pourrait par exemple directement fixer l'extrémité proximale 2a de la tige 2 au dispositif de prise de vue 4 ou à un boîtier de ce dispositif 4.

Quelle que soit la manière avec laquelle ces deux éléments sont rendus solidaires l'un de l'autre, le dispositif de prise de vue 4 est orienté vers l'extrémité distale 2b de la tige flexible 2. Dit autrement, l'orientation du dispositif de prise de vue 4 est telle que l'extrémité distale 2b de la tige flexible 2 est dans son champ de vision, c'est-à-dire dans la zone de l'espace que le capteur ou les capteurs du dispositif de prise de vue 4 perçoit. Comme on l'a mentionné précédemment, le dispositif 1 peut être configuré pour que, au repos, l'extrémité distale 2b soit disposée au centre, ou dans le voisinage du centre, de ce champ de vision.

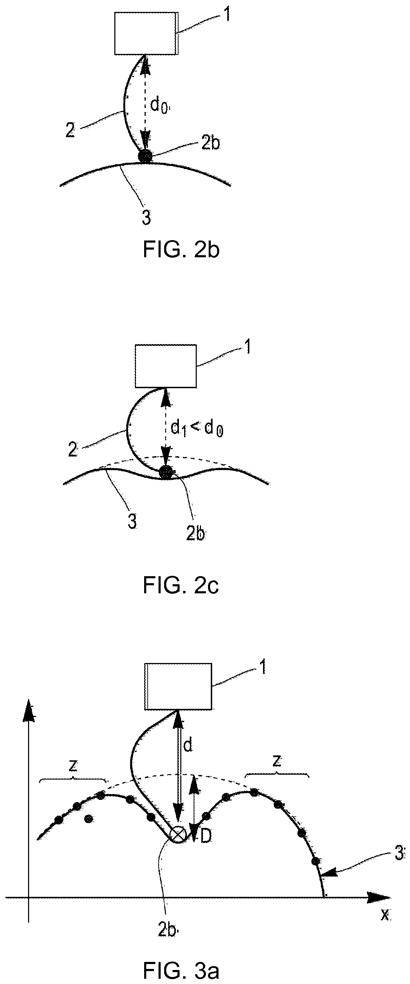

Lorsque le dispositif de mesure 1 applique un effort au corps sous test (comme cela sera présenté en relation avec la description des figures 2), l'orientation du dispositif de prise de vue 4 vers l'extrémité distale 2b de la tige 2 conduit également à faire entrer une portion du corps situé dans le voisinage du point de contact dans le champ de vision du dispositif de prise de vue 4. Ce voisinage du point de contact étant susceptible d'être déformé par l'application de l'effort, la disposition du dispositif de prise de vue 4 permet également de saisir un profil de déformation et une image de la déformation du corps sous test.

Selon l'invention, le dispositif de prise de vue 4 est pourvu d'un capteur apte à fournir une carte de profondeur. Le capteur procède à la mesure dans un plan, appelé « plan image », qui est muni d'un repère (x,y) désigné « repères du dispositif de prise de vue » dans la suite de la présente description. Ce repère peut être augmenté d'une direction de profondeur z, perpendiculaire au plan image afin de former un repère complet (x, y, z). Le dispositif de prise de vue 4 étant solidaire, sans aucun degré de liberté, de la tête 1b du dispositif de mesure 1, le repère du dispositif de prise de vue forme également un repère lié au dispositif de mesure 1.

La carte de profondeur fournie par le dispositif de prise de vue 4 peut prendre la forme d'un nuage de points dont les coordonnées sont établies dans le repère complet (x, y, z). La carte peut alternativement se présenter sous la forme d'une image constituée d'éléments du plan image du capteur respectivement associés à des points de la scène imagée, chaque élément du plan image étant également associé à une information de distance séparant ce plan du dispositif de prise de vue 4 du point de la scène en question.

Avantageusement, mais sans que cela forme une caractéristique essentielle de l'invention, le dispositif de prise de vue 4 peut également fournir une image numérique de son champ de vision. A cet effet, le dispositif de prise de vue 4 peut disposer d'une optique, ou de tout autre moyen, permettant d'imager la surface déformée du corps sous tests ainsi que de l'extrémité distale 2b de la tige 2. On pourra choisir la distance focale de l'optique selon l'étendue du champ de vision souhaitée et donc l'étendue de la surface imagée du corps sous test. On peut également choisir avantageusement une profondeur de champ suffisante pour qu'elle comprenne l'extrémité distale 2b de la tige flexible 2 pour toute la gamme d'efforts applicables sur le corps, et donc pour toute la gamme de flexion de la tige 2.

Par souci de clarté, on rappelle qu'une image numérique est définie par des éléments d'images, désignées « pixels », qui portent au moins une valeur numérique d'intensité lumineuse de niveaux de gris ou de couleur. Les pixels sont généralement disposés sous la forme d'une matrice, en lignes et en colonnes. Une telle image numérique peut toutefois être enregistrée dans le dispositif de prise de vue 4, et être transmise, sous une autre forme, par exemple une forme compressée, mais dans tous les cas l'image peut être manipulée pour représenter une image composée de pixels disposés matriciellement.

Lorsque le dispositif de prise de vue 4 combine à la fois des informations de distance et des informations de couleur ou d'intensité lumineuse, ce dispositif peut par exemple être formé d'une caméra RGB-D, dont on trouve une description dans les documents US20070216894 et US7433024. Outre le capteur d'image traditionnelle, permettant par exemple de déterminer pour chaque pixel une valeur d'intensité de rayonnement dans le domaine du rouge, du vert et du bleu, une caméra RGB-D projette également un rayonnement infrarouge dont la réflexion est capturée par un capteur d'image infrarouge. Le rayonnement mesuré est traité pour déterminer la distance séparant le capteur des éventuels objets disposés dans le champ de vision, selon par exemple la technologie décrite dans les documents précités. Le capteur infrarouge et le capteur d'image sont calibrés entre eux, si bien qu'il est possible d'associer une information de profondeur aux informations d'intensité pour chaque pixel.

On note que les données du dispositif de prise de vue 4 conforme à la présente description sont fournies sous un format quelconque, qui n'est pas limité à celui d'une image numérique dont les pixels sont augmentés d'informations de profondeur. Ces données peuvent également se présenter par exemple, comme on l'a déjà mentionné, sous un format de nuage de points. Dans un tel format, chaque point du nuage est représenté par ses coordonnées dans le repère complet (x, y, z) éventuellement augmenté d'une information de couleur.

Quel que soit le format dans lequel les données établies par le dispositif de prise de vue 4 sont conditionnées, celles-ci seront désignées par les termes « images » pour les données relatives à l'intensité du rayonnement visible perçu sur le plan image du capteur d'image, et par « carte de profondeur » pour les données relatives à la distance séparant les objets de la scène au dispositif de prise de vue 4. Et ces données sont repérées dans un même repère associé au dispositif de prise de vue 4, et donc au dispositif de mesure 1.

Le dispositif de prise de vue 4 peut permettre de prendre une succession d'images et de cartes de profondeur, par exemple toutes les 20 ms, pour constituer une séquence animée. Lorsque le dispositif de mesure 1 est employé pour effectivement analyser la déformation d'un corps, le dispositif de prise de vue 4 fournit des données qui peuvent être représentées sous la forme d'une séquence animée de la déformation de ce corps, et comprend à la fois des images et de cartes de profondeur successives de la déformation.

Pour enclencher la prise d'une image et/ou d'une carte, ou d'une séquence de telles images et de telles cartes, le dispositif de mesure 1 peut être muni d'un interrupteur 5 actionnable par l'opérateur. Celui-ci peut par exemple actionner manuellement l'interrupteur et enclencher la ou les prises de vue, alors qu'il déplace le dispositif de mesure 1 vers le corps sous tests et qu'il applique un effort à ce corps tendant à le déformer. À l'issue de cette étape, on dispose alors d'une séquence animée d'images et de carte de profondeur imageant la déformation du corps. Ces images comprennent bien entendu l'extrémité distale 2b de la tige flexible 2 qui est disposée à tout moment dans le champ de vision du dispositif de prise de vue 4.

Pour des raisons pratiques, on peut prévoir que l'interrupteur 5 se présente sous la forme d'une gâchette disposée sur la poignée de préhension 1a du dispositif 1, comme cela est représenté sur la figure 1. Cela permet à l'opérateur d'utiliser le dispositif de mesure 1 d'une seule main et d'actionner l'interrupteur 5 tout en manipulant le dispositif 1 pour l'appliquer sur le corps sous test.

Les données élaborées par le dispositif de prise de vue 4, images numériques et cartes de profondeur ou toutes autres données élaborées à partir de ces informations, peuvent être communiquées aux moyens de calcul. Les moyens de calcul peuvent à cet égard comprendre un dispositif de calcul distant, pour y être exploitées et une unité de transmission. À cet effet, le dispositif de mesure 1 peut comprendre une unité de transmission 6 reliée au dispositif de prise de vue 4. Cette unité de transmission 6 peut prendre la forme d'un simple connecteur permettant d'enficher un câble de liaison entre le dispositif de mesure 1 le dispositif de calcul. Il peut s'agir alternativement d'un circuit électronique plus complexe permettant de conditionner les données avant de les transmettre, par exemple à l'aide d'une liaison sans fil, au dispositif de calcul. L'unité de transmission peut également permettre de configurer et/ou d'opérer, à partir du dispositif de calcul distant, le dispositif de prise de vue 4 et plus généralement le dispositif de mesure 1.

Les moyens de calcul peuvent également comprendre une unité de calcul solidaire du dispositif de mesure 1. L'unité de calcul peut comprendre, par exemple, un microcontrôleur, une puce graphique, un FPGA, spécifiquement configurés pour réaliser des traitements sur les données fournies par le dispositif de prise de vue 4, avant que ces données traitées ne soient transmises aux dispositifs de calcul distant.

Ces traitements peuvent correspondre à une simple mise en forme des images et des cartes de profondeur fournie par le dispositif de prise de vue 4, par exemple pour les fournir sous la forme d'un nuage de points colorés comme cela a été évoqué précédemment, ou pour en réduire la résolution, ou encore pour en extraire des informations plus riches comme par exemple la force exercée par la tige flexible 2 sur le corps sous test qui fait l'objet du procédé de mesure dont la description suit.

Les figures 2a, 2b et 2c représentent schématiquement le comportement de la tige flexible 2 avant que l'extrémité distale 2b ne contacte un corps sous test 3, au moment du contact de cette extrémité distale 2b avec le corps 3 sans toutefois appliquer d'efforts sur ce corps, et lorsque le dispositif 1 est approché suffisamment près du corps 3 pour qu'un effort s'y applique.

Sur les figures 2a et 2b aucun effort n'est donc appliqué sur le corps 3 et celui-ci présente donc sa forme « naturelle », sans déformation. C'est également le cas de la tige flexible 2. L'extrémité distale 2b est donc séparée du dispositif de mesure 1 d'une distance au repos d0. Cette distance peut être par exemple celle séparant l'extrémité distale 2b de l'extrémité proximale 2a de la tige flexible 2, ou de tout autre point fixe du dispositif de mesure 1.

Sur la figure 2c, le dispositif de mesure 1 est approché suffisamment du corps 3 pour que la tige 2 applique une force sur ce corps 3 au niveau du point de contact. Le corps 3 est déformé au point de contact et dans son voisinage. La tige 2 étant flexible, celle-ci est également déformée en flexion, si bien que la distance d1 séparant son extrémité distale 2b de son extrémité proximale 2a est réduite, par rapport à la distance au repos d0 des figures 2a et 2b.

On note que, connaissant la distance séparant l'extrémité distale 2b du dispositif de mesure 1, on peut déterminer de manière univoque l'effort appliqué par la tige 2 (et donc par le dispositif de mesure 1) sur ce corps 3.

Dans l'illustration des figures 2a à 2c, le dispositif de mesure 1 est approché du corps 3 pour appliquer une force, par l'intermédiaire de la tige 2, qui n'a pas de composante tangentielle. D'une manière plus générale, le dispositif de mesure 1 peut être rapproché du corps 3 selon des directions variées afin d'impartir au corps 3 un effort présentant une composante tangentielle. Dans ce cas, l'extrémité distale 2b de la tige flexible 2 se déplace non seulement selon une direction de profondeur z dans le repère complet lié au dispositif, mais également selon des directions perpendiculaires à cette direction de profondeur z. Dit autrement, les coordonnées de l'extrémité distale 2b de la tige 2 sont susceptibles de varier selon les trois composantes (x, y, z) dans un repère lié au dispositif de mesure 1 lorsqu'on déplace celui-ci pour appliquer un effort sur le corps à l'aide de la tige flexible 2.

Dans cette approche générale, on note qu'il est possible de déterminer toutes les composantes de la force appliquée au corps 3 par le dispositif de mesure 1 à partir de la position de l'extrémité distale 2b de la tige 2 dans le repère lié au dispositif de mesure 1.

Dans certains cas simples, la relation reliant la position de l'extrémité distale 2b à cette force peut être formulée explicitement, à partir par exemple de la dimension de la tige 2 et/ou de coefficients de raideurs. Mais plus généralement, cette relation peut être établie par une étape préalable de calibration au cours de laquelle on applique le dispositif de mesure 1 sur un capteur d'efforts pré-calibré selon 3 composantes (dit capteur « tri-axes »).

Par exemple, le capteur peut être porté par un plateau rigide que l'on peut déplacer de manière contrôlée selon 3 directions orthogonales. On dispose fixement le dispositif de mesure 1 vis-à-vis du plateau et au droit du capteur d'efforts de sorte que l'extrémité distale 2b de la tige flexible 2 contacte ce capteur. On relève ensuite une batterie de mesures en déplaçant le plateau rigide selon les 3 directions possibles de manière à collecter :
- les composantes fx, fy, fz de la force appliquée par la tige flexible 2 sur le capteur, et telles que ces composantes sont fournies par le capteur ;
- les coordonnées (x, y, z) de la partie distale 2b de la tige 2 dans le repère lié au dispositif de prise de vue 4. Ces coordonnées peuvent être relevées manuellement à partir de la carte de profondeur fournie par ce dispositif 4 ou par traitement numérique de cette carte, comme cela sera explicité dans une autre partie de cette description.

On peut de la sorte collecter des couples de mesures {(x, y, z) ; (fx, fy, fz)} reliant la position (x, y, z) de l'extrémité distale 2b de la tige 2 aux composantes fx, fy, fz de la force appliquée par cette tige 2. Ces couples de mesures peuvent être exploités pour déterminer, par interpolation, les composantes de la force appliquée par le dispositif de mesure 1 sur le corps pour toute position de l'extrémité distale 2b dans un repère lié au dispositif de mesure 1 (comme celui associé au dispositif de prise de vue 4).

En d'autres termes, dans un dispositif de mesure 1 conforme à l'invention, la position de l'extrémité distale 2b de la tige 2 vis-à-vis d'un point de référence du dispositif de mesure 1 peut être reliée de manière univoque aux composantes de la force appliquée par ce dispositif 1 sur un corps 3.

Cette caractéristique du dispositif de mesure 1 est exploitée afin de fournir un procédé de mesure de la force exercée sur un corps 3 particulièrement simple à mettre en œuvre, qui fait l'objet de la section suivante de la présente description.

### Procédé de mesure de la force exercée sur un corps

Le procédé de mesure comprend une première étape d'application d'un dispositif de mesure 1, tel que décrit précédemment, sur un corps sous test 3. Comme on l'a déjà évoqué, au cours de cette étape on approche le dispositif de mesure 1 du corps 3 pour mettre en contact forcé sur ce corps 3 l'extrémité distale 2b de la tige flexible 2. On exerce donc une force sur le corps 3, cette force tendant à déformer le corps 3 au point de contact et dans son voisinage. On déforme également la tige flexible 2. Cette étape peut être mise en œuvre par un opérateur ou, alternativement, par un système automatisé, tel qu'un bras de robot dont l'extrémité serait équipée du dispositif de mesure 1.

Une fois que le contact forcé du dispositif de mesure 1 sur le corps 3 a été établi, le dispositif 1 peut être éloigné du corps 3 et on peut interrompre le procédé, ou le répéter en un point de contact identique ou distinct.

Le procédé de mesure comprend également une étape d'acquisition au cours de laquelle on procède à l'acquisition d'au moins une carte de profondeur, en un instant de mesure, à l'aide du dispositif de prise de vue 4. Comme on l'a déjà mentionné, on procédera préférentiellement à l'acquisition d'une pluralité de cartes en des instants de mesures successifs, de manière à collecter une séquence animée de la déformation du corps sous test 3 provoqué par les forces exercées par le dispositif de mesure 1 au cours du temps.

L'étape d'acquisition peut être déclenchée et interrompue par l'interrupteur 5 ou par la gâchette du dispositif de mesure 1 opéré par l'opérateur au cours de l'étape d'application. Alternativement, l'acquisition peut être déclenchée par le dispositif de calcul distant auquel le dispositif de mesure 1 peut être relié, notamment lorsque celui-ci fait partie d'un système de mesure automatisé.

Quelle que soit la manière selon laquelle on déclenche l'étape d'acquisition, on fera en sorte que celle-ci soit temporellement incluse ou chevauchant l'étape d'application du dispositif de mesure 1, de sorte que l'on puisse procéder à l'acquisition d'au moins une carte de profondeur du corps alors que la tige flexible est en contact forcé avec ce corps.

Lorsque le dispositif de prise de vue 4 le permet, cette étape d'acquisition procède également à l'acquisition d'images numériques du corps 3 simultanément à l'acquisition des cartes de profondeurs.

Les données dont on fait l'acquisition au cours de cette étape peuvent être stockées dans une mémoire tampon du dispositif de prise de vue 4 ou du dispositif de mesure 1. Elles peuvent être traitées par l'unité de calcul et/ou transmises au dispositif de calcul distant en vue d'y être traitées par l'intermédiaire de l'unité de transmission 6 auquel le dispositif de prise de vue 4 est directement ou indirectement relié.

Que ces données soient traitées localement ou par le dispositif de calcul distant, un procédé de mesure conforme à l'invention comprend une étape de traitement de ces données (au moins une carte de profondeur et, éventuellement, au moins une image numérique) visant à établir l'effort exercé par le dispositif 1 sur le corps 3, à l'instant de la mesure.

À cet effet, l'étape de traitement comprend une première sous-étape visant à repérer, par une technique de traitement numérique, l'extrémité distale 2b de la tige flexible 2 dans la carte de profondeur ou, lorsque celle-ci est disponible, dans l'image numérique. Cette sous-étape peut être facilitée par la présence d'un embout de protection présentant une forme spécifique, permettant de bien distinguer son contour dans la carte de profondeur. Cette sous-étape de repérage peut comprendre un traitement numérique classique dans le domaine de la vision par ordinateur et par exemple être mise en œuvre par exemple par un réseau de neurones. Cette sous-étape fournit, à l'issue de son exécution, les coordonnées, dans le repère (x, y, z) associé au dispositif de prise de vue 4, de l'extrémité distale 2b de la tige 2.

Lorsque ce traitement se fait sur l'image numérique, l'embout peut être choisi pour présenter également une couleur spécifique facilitant ainsi son repérage sur l'image. Le repérage de l'embout peut très simplement s'exprimer sous la forme des rangs de colonne et de ligne d'un pixel de l'image. On utilise alors les rangs du pixel et la carte de profondeur pour déterminer les coordonnées, dans le repère (x, y, z) associé au dispositif de prise de vue 4, de l'extrémité distale 2b de la tige 2.

Si la carte de profondeur présente une résolution moindre que celle de l'image, cette sous-étape peut comprendre ou correspondre à la recherche du point de la carte de profondeur le plus proche des coordonnées repérées dans la sous-étape précédente, afin de prélever dans la carte de profondeur une information cohérente.

Dans tous les cas, et quel que soit le traitement mis en œuvre cours de cette sous-étape, on dispose l'issue de celle-ci des coordonnées, dans le repère (x, y, z) associé au dispositif de prises de vue 4, de l'extrémité distale 2b de la tige 2, à l'instant de la mesure. Lorsque l'on procède à plusieurs mesures successives, on dispose d'une liste de ces coordonnées.

Dans une deuxième sous-étape de l'étape de traitement, on convertit la position de l'extrémité distale 2b de la tige 2 à l'instant de la mesure en une force. Pour faire cette conversion, on exploite la fonction ou la table de calibration de la tige flexible 2 associant la position de l'extrémité distale 2b à une force appliquée. Comme on l'a vu préalablement, cette fonction ou cette table peut avoir été élaborée dans une étape de calibration, préalable aux étapes composant le procédé de mesure lui-même.

On a ainsi illustré sur les figures 3a à 3c un exemple de mise en œuvre des traitements qui viennent d'être décrits. Dans cet exemple, le dispositif de prise de vue 4 correspond à une caméra RGB-D et on exploite les informations de couleur fournies par les images numériques pour identifier l'embout de la tige flexible 2. On a représenté sur la figure 3a le dispositif de mesure 1 en contact forcé avec un corps 3 au niveau de l'extrémité distale 2b de la tige flexible 2.

Pour simplifier la représentation, le corps sous test 3 présente ici une seule dimension, et une image de ce corps est donc composée de pixels x1 à xn disposés en ligne selon un axe X qui forme le repère du dispositif de prise de vue 4. Chaque pixel xi est, dans cet exemple, associé à une mesure d'intensité de couleur rouge, fournie par un capteur d'image du dispositif de prise de vue 4, et à une mesure de profondeur z, fournie par exemple par un dispositif à capteur d'image infrarouge.

Dans l'exemple représenté, on considère que le corps 3 présente une surface exposée essentiellement de couleur bleue, ce qui contraste bien avec l'embout rouge de la tige flexible 2 et ce qui facilite le repérage de cet embout dans l'image.

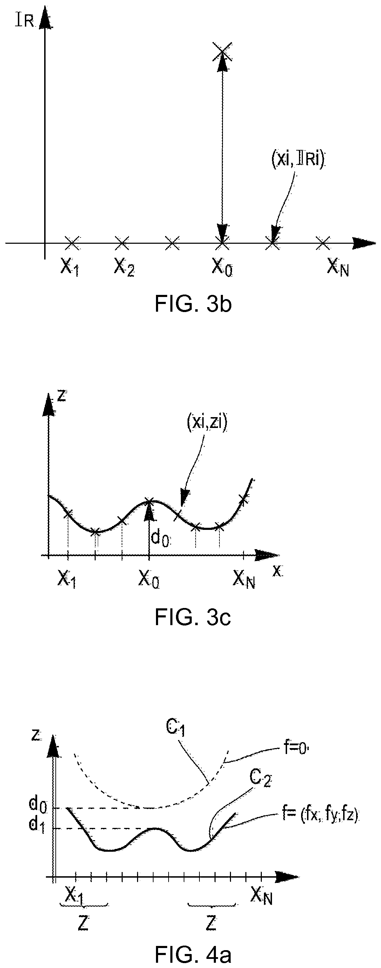

L'illustration de la figure 3c représente la carte de profondeur, ici la distance zi associée à chaque pixel xi, et l'illustration de la figure 3b l'image d'intensité I_{R} de rouge, c'est-à-dire l'intensité de couleur rouge I_{Ri} associée à chaque pixel xi.

La première sous-étape de repérage est facilitée par le choix de la couleur très distincte de l'extrémité distale 2b de la tige 2 en comparaison avec celle du corps 3, si bien que sur l'image composée de pixels associés à une intensité de couleur rouge, il est facile de repérer le pixel x0 dont l'intensité dans le rouge est bien marquée vis-à-vis des autres pixels qui sont eux en correspondance au corps sous test 3, ici de couleur bleue. Dans la pratique on pourra mettre en œuvre des algorithmes issus du domaine de la vision par ordinateur pour repérer dans l'image l'extrémité distale 2b de la tige 2, et pour choisir un pixel représentatif de la position de cette extrémité dans l'image.

Connaissant le pixel x0 (ou plus généralement les coordonnées dans le repère associé au dispositif de prise de vue 4) correspondant de l'extrémité distale 2b, il est aisé de déterminer à partir de la carte de profondeur la distance mesurée d0 associée à ce pixel, à partir de la carte de profondeur illustrée sur la figure 3c. Ensuite on convertit les informations de pixels et de distance pour déterminer les coordonnées de l'extrémité distale 2b de la tige 2, que l'on convertit pour obtenir la force appliquée au corps 3 à l'instant de mesure, c'est-à-dire à l'instant à laquelle l'image et la carte de profondeur ont été acquises. Si l'on a procédé à l'acquisition d'une pluralité d'images et de cartes de profondeur, on pourra répéter les sous-étapes sur chacune des instances de la pluralité, et déterminer la pluralité d'efforts correspondants.

On notera que les informations fournies par la carte de profondeur, ici, pour chaque pixel de l'image numérique, la distance séparant la surface exposée du corps 3 au dispositif de prise de vue 4, est la combinaison de deux phénomènes : la déformation du corps 3 qui tend à éloigner sa surface du dispositif de mesure 1 et la déformation de la tige 2 qui tend à rapprocher le dispositif de mesure 1 du corps 3. Toutefois, l'estimation de la force appliquée ne dépend que de cette dernière distance (ou plus précisément de la position (x, y, z) de l'extrémité distale 2b de la tige 2 vis-à-vis du dispositif de mesure 1), si bien que le procédé peut estimer cette force sans équivoque.

### Procédé de caractérisation de la rigidité d'un corps

Pour être complet toutefois, on note que les informations fournies par une seule mesure (i.e. une carte de profondeur et, éventuellement, une image numérique) ne sont pas suffisantes pour caractériser à elles seules la rigidité du corps sous test 3 au niveau du point de contact.

En préambule, on rappelle que la rigidité d'un corps peut être caractérisé de multiples manières. Elle peut par exemple être représentée par la distance de déformation du corps au niveau du point de contact, selon la force appliquée, par exemple d'intensités croissantes ou d'orientations différentes. Par « distance de déformation », on désigne la distance D entre le point de contact lorsqu'aucune force n'est appliquée et ce point de contact lorsqu'une force déterminée est appliquée par le dispositif de mesure 1, tel que cela est rendu visible sur la figure 2a.

Alternativement, cette déformation peut être caractérisée et représentée par un profil de déformation (par exemple établi à partir des points (xi, zi) de la carte de profondeur, pour reprendre l'exemple des figures 3a à 3c) du corps 3 au voisinage du point de contact, plutôt que par une simple distance de déformation D au niveau du point de contact.

Quelle que soit la manière avec laquelle la rigidité du corps 3 est caractérisée, elle repose sur la disponibilité d'une pluralité de cartes de profondeur, permettant d'évaluer la déformation du corps 3 pour au moins deux forces appliquées différentes. Avantageusement, il s'agit d'une première carte correspondant à la forme naturelle du corps 3 (en l'absence d'application de toute force) et d'au moins une deuxième carte correspondant à la forme du corps 3 lorsque le dispositif de mesure 1 applique au moins une force déterminée, selon ses 3 composantes fx, fy, fz.

A titre d'exemple on a représenté sur la figure 4a en trait pointillé la carte de profondeur C1 élaborée par le dispositif de mesure 1 de la figure 2a lorsque l'extrémité distale 2b de la tige flexible 2 est en contact avec le corps 3, sans toutefois exercer d'effort sur ce corps (force appliquée nulle). La carte de profondeur définit donc le profil non déformé de ce corps 3, dans le champ de vision du dispositif de prise de vue 4. On retrouve sur cette carte de profondeur, au niveau du point de contact entre la tige 2 et le corps 3, la distance d0, correspondant à la distance séparant l'extrémité distale 2b de la tige 2 de la tête 1b du dispositif de mesure 1, lorsque cette tige 2 n'est pas fléchie.

Sur cette même figure 4a, on a représenté en trait plein la carte de profondeur C2 élaborée par le dispositif de mesure lorsque l'extrémité distale 2b de la tige flexible 2 exerce un effort sur le corps 3, tel que cela est représenté sur la figure 2a. La tige 2 étant fléchie, la distance d1 séparant l'extrémité 2b de la tige 2 et le corps 3 est réduite, inférieure à la distance d0. Par ailleurs, le dispositif de mesure 1 s'étant globalement approché du corps sous test 3 pour y appliquer une force de déformation, la carte de profondeur C2 présente des grandeurs généralement plus petites que celles de la carte de profondeur C1 du corps non déformé. L'application du procédé de mesure précédemment décrit permet d'identifier la force appliquée sur le corps selon ses trois composantes (fx, fy, fz).

On observe sur la figure 2a que le corps 3 est déformé dans le voisinage du point de contact, mais que ce n'est pas le cas dans des zones périphériques Z de ce voisinage. Toutefois, un décalage est introduit entre les cartes de profondeurs représentées sur la figure 4a.

Aussi, au cours d'un procédé de caractérisation conforme à l'invention, les profils de déformation du corps 3 établis à l'aide de la première carte et de la deuxième carte de profondeur (ou cette première et deuxième carte directement) sont recalés l'un avec l'autre. Plus généralement, toutes les cartes de profondeurs C1, C2 qui ont été acquises au cours du procédé sont recalées les unes avec les autres. De la sorte, on recale entre eux les profils élaborés par le dispositif de prise de vue 4 porté par la tête 1b du dispositif de mesure 1.

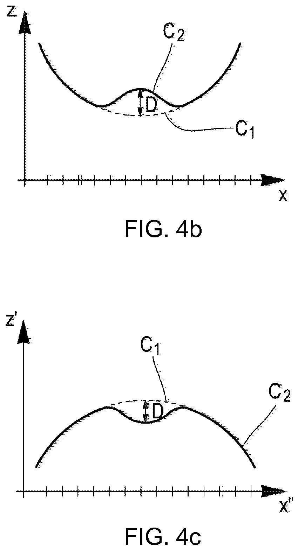

Cette étape de recalage peut consister à numériquement chercher à faire correspondre respectivement entre elles les zones non déformées des cartes de profondeurs C1, C2, par exemple au moins une zone périphérique Z de la figure 4a. Une zone non déformée est suffisamment éloignée du point de contact pour que son décalage d'une carte à l'autre ne soit pas dû à la déformation du corps 3, mais seulement au déplacement du dispositif de mesure 1. La figure 4b représente les deux cartes de profondeurs C1, C2 de la figure 4a, après application de cette étape de recalage. La figure 4c représente les cartes de profondeur C1, C2 replacée dans un repère (x', z') lié au corps sous test 3, pour présenter le profil non déformé et déformé du corps 3.

Pour faciliter cette étape de recalage des cartes de profondeurs, on peut prévoir de munir le corps 3 de repères visuels, désignés repères de recalage, qui peuvent être identifiés sur les images numériques fournies par le dispositif de prise de vue 4. Ces repères peuvent notamment être utiles pour détecter une déformation présentant une composante en rotation du corps 3, qui ne serait pas détectable sur les cartes de profondeurs.

Une fois les cartes de profondeurs recalées les unes aux autres, il est possible de caractériser la rigidité du corps 3, par exemple en identifiant la distance de déformation D ou le profil de déformation (qui apparaît sur les cartes de profondeurs de la figure 4a et sur les cartes de profil sur la figure 4c), cette distance D ou ce profil étant associés à la force déterminée f appliquée sur le corps. A titre d'exemple complémentaire de la caractérisation de la rigidité du corps 3, on peut fournir le vecteur de déplacement du point de contact de la tige 2 sur le corps 3, ce vecteur étant élaboré en prenant la différence entre les coordonnées de l'extrémité distale 2b de la tige 2 dans les deux cartes de profondeur recalées. Le vecteur peut être associé à la force appliquée déterminée au cours du procédé de mesure, pour caractériser pleinement le corps 3.

Bien entendu l'invention n'est pas limitée au mode de mise en œuvre décrit et on peut y apporter des variantes de réalisation sans sortir du cadre de l'invention tel que défini par les revendications.

En particulier, le système et les procédés de mesure et de caractérisation qui viennent d'être décrits peuvent être exploités pour caractériser la rigidité d'un corps de toute nature. Il peut notamment s'agir d'un corps humain, mais sans que cette application particulière soit limitative. Il peut également s'agir de tout objet dont on souhaite par exemple caractériser la rigidité.

Le dispositif et le procédé peuvent trouver de nombreuses applications pratiques, par exemple pour élaborer un modèle numérique de l'objet caractérisé, ce modèle permettant de représenter ou de simuler la déformation d'un objet ou d'un corps lorsque celui-ci est soumis à des efforts extérieurs.

## Revendications

1. Système de mesure (10) destiné à mesurer une force exercée sur un corps (3), le système de mesure comprend un dispositif de mesure (1) et des moyens de calcul,
le dispositif de mesure (1) comprend :
- une tige flexible (2) présentant une extrémité proximale (2a) et une extrémité distale (2b) destinée à entrer en contact avec le corps (3) et à appliquer une force tendant à le déformer au voisinage d'un point de contact, la tige flexible (2) étant configurée pour fléchir dès lors qu'elle applique une force par son extrémité distale (2b) sur le corps, la tige flexible est courbée ou présente un coude ;
- un dispositif de prise de vue (4) solidaire de l'extrémité proximale (2a) et orientée vers l'extrémité distale (2b) de la tige flexible (2) pour fournir au moins une carte de profondeur du corps (3) au voisinage de l'extrémité distale (2b) de la tige flexible (2), et de l'extrémité distal (2b) ;
les moyens de calcul (7) sont configurés pour, à partir de la carte de profondeur de l'extrémité distale de la tige, déterminer la force exercée par la tige flexible (2) par son extrémité distale sur le corps.

2. Système de mesure (10) selon la revendication 1, dans lequel la tige flexible (2) est configurée pour appliquer une force comprise entre 0,1 N et 10 N.

3. Système de mesure (10) selon l'une des revendications précédentes, lequel le dispositif de mesure (1) comprend un interrupteur (5) pour actionner le dispositif de prise de vue (4).

4. Système de mesure (10) selon l'une des revendications précédentes comprenant en outre une poignée de préhension (1a) solidaire du dispositif de prise de vue (4) et de la tige flexible (2).

5. Système de mesure (10) selon les revendications 3 et 4 dans lequel l'interrupteur (5) se présente sous la forme d'une gâchette.

6. Système de mesure (10) selon l'une des revendications précédentes, les moyens de calcul comprennent une unité de transmission (6), et un dispositif de calcul distant, l'unité de transmission étant configurée pour échanger des données avec un dispositif de calcul distant.

7. Système de mesure (10) selon l'une des revendications 1 à 6, dans lequel les moyens de calcul comprennent une unité de calcul configurée pour traiter la carte de profondeur.

8. Système de mesure (10) selon l'une des revendications précédentes dans lequel le dispositif de prise de vue (4) fournit également une image numérique du corps (3) au voisinage de l'extrémité distale (2b) de la tige flexible (2).

9. Système de mesure (10) selon la revendication précédente dans lequel l'extrémité distale (2b) comporte un embout présentant une couleur pour être facilement repérée sur l'image fournie par le dispositif de prise de vue (4).

10. Système de mesure (10) selon l'une des revendications précédentes dans lequel l'extrémité distale (2b) comporte un embout présentant une forme choisie pour être facilement repérée sur la carte fournie par le dispositif de prise de vue (4).

11. Procédé de mesure d'une force exercée sur un corps (3), le procédé comprenant :
- une étape d'application du système de mesure (10) selon l'une des revendications précédentes pour que l'extrémité distale (2b) de la tige (2) contacte le corps (3) en un point de contact et applique une force tendant à déformer le corps (3) au voisinage du point de contact ;
- une étape d'acquisition, à un instant de mesure, d'au moins une carte de profondeur au cours de l'étape d'application ;
- une étape de traitement de la carte de profondeur pour établir la force exercée sur le corps (3).

12. Procédé de mesure d'une force exercée sur un corps (3) selon la revendication précédente dans lequel l'étape de traitement comprend une sous-étape de repérage de l'extrémité distale (2b) de la tige (2) dans la carte de profondeur pour fournir des coordonnées de cette extrémité (2b) dans un repère associé au dispositif de mesure (1).

13. Procédé de mesure d'une force exercée sur un corps (3) selon l'une des revendications 11 à 12 comprenant une étape préalable de calibration du dispositif de mesure (1).

14. Procédé de caractérisation de la rigidité d'un corps (3) comprenant l'acquisition, au cours d'un procédé de mesure conforme à l'une des revendications 11 à 13, d'une pluralité de cartes de profondeur en des instants de mesures successifs avec un système de mesure (10) selon l'une des revendications 1 à 10.

15. Procédé de caractérisation de la rigidité d'un corps (3) selon la revendication précédente, comprenant une étape de recalage de la pluralité de cartes de profondeur.

16. Procédé de caractérisation de la rigidité d'un corps (3) selon la revendication précédente dans lequel le corps (3) comprend des repères de recalage permettant de recaler la pluralité de cartes de déformation.

## Patentansprüche

1. Messsystem (10), das zum Messen einer Kraft, die auf einen Körper (3) ausgeübt wird, bestimmt ist, wobei das Messsystem eine Messvorrichtung (1) und Berechnungsmittel umfasst, wobei die Messvorrichtung (1) umfasst:
- einen flexiblen Stab (2), der ein proximales Ende (2a) und ein distales Ende (2b) aufweist, der dazu bestimmt ist, mit dem Körper (3) in Kontakt zu kommen und eine Kraft auszuüben, die dazu tendiert, ihn in der Nähe eines Kontaktpunkts zu verformen, wobei der flexible Stab (2) konfiguriert ist, um sich zu biegen, sobald er durch sein distales Ende (2b) eine Kraft auf den Körper ausübt, wobei der flexible Stab gekrümmt ist oder eine Biegung aufweist;
- eine Bildaufnahmevorrichtung (4), die an dem proximalen Ende (2a) und dem distalen Ende (2b) fest verbunden und auf das distale Ende (2b) des flexiblen Stabs (2) ausgerichtet ist, zum Bereitstellen mindestens einer Tiefenkarte des Körpers (3) in der Nähe des distalen Endes (2b) des flexiblen Stabs (2);
wobei die Berechnungsmittel (7) konfiguriert sind, um aus der Tiefenkarte des distalen Endes des Stabs die Kraft zu bestimmen, die durch den flexiblen Stab (2) durch sein distales Ende auf den Körper ausgeübt wird.

2. Messsystem (10) nach Anspruch 1, wobei der flexible Stab (2) zum Ausüben einer Kraft, die zwischen 0,1 N und 10 N liegt, konfiguriert ist.

3. Messsystem (10) nach einem der vorstehenden Ansprüche, wobei die Messvorrichtung (1) einen Schalter (5) zum Betätigen der Bildaufnahmevorrichtung (4) umfasst.

4. Messsystem (10) nach einem der vorstehenden Ansprüche, ferner umfassend einen Griff zum Greifen (1a), der mit der Bildaufnahmevorrichtung (4) und dem flexiblen Stab (2) fest verbunden ist.

5. Messsystem (10) nach den Ansprüchen 3 und 4, wobei sich der Schalter (5) in der Form eines Auslösers darstellt.

6. Messsystem (10) nach einem der vorstehenden Ansprüche, wobei die Berechnungsmittel eine Übertragungseinheit (6) und eine entfernte Berechnungsvorrichtung umfassen, wobei die Übertragungseinheit zum Datenaustauschen mit einer entfernten Berechnungsvorrichtung konfiguriert ist.

7. Messsystem (10) nach einem der Ansprüche 1 bis 6, wobei die Berechnungsmittel eine Berechnungseinheit, die zum Bearbeiten der Tiefenkarte konfiguriert ist, umfassen.

8. Messsystem (10) nach einem der vorstehenden Ansprüche, wobei die Bildaufnahmevorrichtung (4) auch ein digitales Bild des Körpers (3) in der Nähe des distalen Endes (2b) des flexiblen Stabs (2) bereitstellt.

9. Messsystem (10) nach dem vorstehenden Anspruch, wobei das distale Ende (2b) ein Endstück umfasst, das eine Farbe zum leichten Auffinden auf dem Bild aufweist, das durch die Bildaufnahmevorrichtung (4) bereitgestellt wird.

10. Messsystem (10) nach einem der vorstehenden Ansprüche, wobei das distale Ende (2b) ein Endstück umfasst, das eine Form aufweist, die zum leichten Auffinden auf der Karte gewählt ist, die durch die Bildaufnahmevorrichtung (4) bereitgestellt wird.

11. Verfahren zum Messen einer Kraft, die auf einen Körper (3) ausgeübt wird, das Verfahren umfassend:
- einen Schritt zum Anwenden des Messsystems (10) nach einem der vorstehenden Ansprüche, so dass das distale Ende (2b) des Stabs (2) den Körper (3) an einem Kontaktpunkt berührt und eine Kraft ausübt, die dazu tendiert, den Körper (3) in der Nähe des Kontaktpunkts zu verformen;
- einen Schritt zum Erfassen, zu einem Messzeitpunkt, mindestens einer Tiefenkarte während des Anwendungsschritts;
- einen Schritt zum Bearbeiten der Tiefenkarte zum Ermitteln der Kraft, die auf den Körper (3) ausgeübt wird.

12. Verfahren zum Messen einer Kraft, die auf einen Körper (3) ausgeübt wird, nach dem vorstehenden Anspruch, wobei der Bearbeitungsschritt einen Unterschritt des Auffindens des distalen Endes (2b) des Stabs (2) in der Tiefenkarte zum Bereitstellen von Koordinaten dieses Endes (2b) in einem Koordinatensystem, das der Messvorrichtung (1) zugeordnet ist, umfasst.

13. Verfahren zum Messen einer Kraft, die auf einen Körper (3) ausgeübt wird, nach einem der Ansprüche 11 bis 12, umfassend einen vorherigen Schritt zum Kalibrieren der Messvorrichtung (1).

14. Verfahren zum Kennzeichnen der Steifigkeit eines Körpers (3), umfassend das Erfassen, im Verlauf eines Verfahrens zum Messen nach einem der Ansprüche 11 bis 13, einer Vielzahl von Tiefenkarten zu aufeinanderfolgenden Messzeitpunkten mit einem Messsystem (10) nach einem der Ansprüche 1 bis 10.

15. Verfahren zum Kennzeichnen der Steifigkeit eines Körpers (3) nach dem vorstehenden Anspruch, umfassend einen Schritt zum Korrigieren der Vielzahl von Tiefenkarten.

16. Verfahren zum Kennzeichnen der Steifigkeit eines Körpers (3) nach dem vorstehenden Anspruch, wobei der Körper (3) Korrekturmarkierungen, die ein Durchfallenlassen der Vielzahl von Verformungskarten ermöglichen, umfasst.

## Claims

1. Measuring system (10) for measuring a force exerted on a body (3), the measuring system comprising a measuring device (1) and computing means, wherein the measuring device (1) comprises:
- a flexible rod (2) having a proximal end (2a) and a distal end (2b) intended to come into contact with the body (3) and to apply a force tending to deform it in the vicinity of a point of contact, the flexible rod (2) being configured to bend as soon as it applies a force via its distal end (2b) to the body, wherein the flexible rod is curved or has a bend;
- an image capturing device (4) rigidly connected to the proximal end (2a) and oriented toward the distal end (2b) of the flexible rod (2), for providing at least one depth map of the body (3) in the vicinity of the distal end (2b) of the flexible rod(2), and of the distal end (2b);
wherein the computing means (7) are configured, from the depth map of the distal end of the rod, to determine the force exerted by the flexible rod (2) by its distal end on the body.

2. Measuring system (10) according to claim 1, wherein the flexible rod (2) is configured to apply a force between 0.1 N and 10 N.

3. Measuring system (10) according to either of the preceding claims, wherein the measuring device (1) comprises a switch (5) to actuate the image capturing device (4).

4. Measuring system (10) according to any of the preceding claims, further comprising a gripping handle (1a) rigidly connected to the image capturing device (4) and to the flexible rod (2).

5. Measuring system (10) according to claims 3 and 4, wherein the switch (5) is in the form of a trigger.

6. Measuring system (10) according to any of the preceding claims, wherein the computing means comprise a transmission unit (6) and a remote computing device, the transmission unit being configured to exchange data with a remote computing device.

7. Measuring system (10) according to any of claims 1 to 6, wherein the computing means comprise a computing unit configured to process the depth map.

8. Measuring system (10) according to any of the preceding claims, wherein the image capturing device (4) also provides a digital image of the body (3) in the vicinity of the distal end (2b) of the flexible rod (2).

9. Measuring system (10) according to the preceding claim, wherein the distal end (2b) comprises a tip having a color so as to be easily identified in the image provided by the image capturing device (4).

10. Measuring system (10) according to any of the preceding claims, wherein the distal end (2b) comprises a tip having a shape chosen to be easily identified on the map provided by the image capturing device (4).

11. Method for measuring a force exerted on a body (3), the method comprising:
- a step of applying the measuring system (10) according to any of the preceding claims so that the distal end (2b) of the rod (2) contacts the body (3) at a contact point and applies a force tending to deform the body (3) in the vicinity of the contact point;
- a step of acquiring, at a measurement time, at least one depth map during the application step;
- a step of processing the depth map to establish the force exerted on the body (3).

12. Method for measuring a force exerted on a body (3) according to the preceding claim, wherein the processing step comprises a sub-step of locating the distal end (2b) of the rod (2) in the depth map to provide coordinates of this end (2b) in a coordinate system associated with the measuring device (1).

13. Method for measuring a force exerted on a body (3) according to any of claims 11 to 12, comprising a preliminary step of calibrating the measuring device (1).

14. Method for characterizing the rigidity of a body (3) comprising acquiring, during a measuring method according to any of claims 11 to 13, a plurality of depth maps at successive measurement times with a measuring system (10) according to any of claims 1 to 10.

15. Method for characterizing the rigidity of a body (3) according to the preceding claim, comprising a step of readjusting the plurality of depth maps.

16. Method for characterizing the rigidity of a body (3) according to the preceding claim, wherein the body (3) comprises readjusting markers allowing the plurality of deformation maps to be readjusted.
